(19) Europäisches Patentamt
European Patent Office
Office européen des brevets

(11) **EP 2 229 665 B1**

(12) **FASCICULE DE BREVET EUROPEEN**

(45) Date de publication et mention de la délivrance du brevet:
**07.03.2012 Bulletin 2012/10**

(51) Int Cl.:
**G08B 21/04** *(2006.01)*    **A61B 5/00** *(2006.01)*
**G06F 19/00** *(2011.01)*

(21) Numéro de dépôt: **08856967.8**

(22) Date de dépôt: **03.12.2008**

(86) Numéro de dépôt international:
**PCT/EP2008/066736**

(87) Numéro de publication internationale:
**WO 2009/071598 (11.06.2009 Gazette 2009/24)**

(54) **PROCEDE ET EQUIPEMENT DE DETECTION DE SITUATION CRITIQUE D'UN SUJET**

VERFAHREN UND VORRICHTUNG ZUM DETEKTIEREN EINER KRITISCHEN SITUATION EINES SUBJEKTS

METHOD AND APPARATUS FOR DETECTING A CRITICAL SITUATION OF A SUBJECT

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **06.12.2007 FR 0759618**

(43) Date de publication de la demande:
**22.09.2010 Bulletin 2010/38**

(73) Titulaires:
• **Vigilio**
  **91000 Evry (FR)**
• **Universite Joseph Fourier - Grenoble 1**
  **38400 St. Martin d'Heres (FR)**

(72) Inventeurs:
• **NOURY, Norbert**
  **F-38000 Grenoble (FR)**
• **LUNDY, Jean-Eric**
  **F-94320 Thiais (FR)**

(74) Mandataire: **Le Forestier, Eric et al**
**Cabinet Regimbeau**
**20, rue de Chazelles**
**75847 Paris Cedex 17 (FR)**

(56) Documents cités:
**WO-A-2006/134041**    **FR-A- 2 808 609**
**US-A1- 2006 097 879**

**Description**

La présente invention concerne un procédé et un dispositif pour détecter une situation critique d'un sujet.

La détection d'une situation critique d'un sujet peut s'avérer utile dans de nombreux cas, par exemple dans le cas des personnes âgées ou malades. Ainsi, les personnes âgées vivent souvent seules et, en cas d'accident tel qu'une chute, elles peuvent être incapables de demander du secours. Il est également possible que ces personnes aient un problème de santé durant leur vie quotidienne ou durant leur sommeil. Il est donc utile de prévoir des systèmes de détection automatique de situation critique pouvant être couplés à des systèmes d'alarme et/ou d'appel.

Il existe des capteurs de chocs portés par des personnes, qui donnent l'alarme s'ils sont soumis à un choc. Cependant, pour qu'un tel système de détection soit efficace, la personne devrait porter un grand nombre de ces capteurs, en principe un sur chaque partie saillante, ce qui n'est pas réalisable en pratique. Il existe également des capteurs de verticalité, qui détectent si une personne est couchée ou debout. Cependant, avec ce type de capteurs, il est impossible de savoir si une personne est allongée de façon volontaire (repos, sieste) ou si elle a été victime d'une chute. La performance d'un capteur de chute ne peut ainsi être appréciée que par les facteurs de sensibilité (capacité du système à détecter *toutes* les chutes) et de spécificité (capacité du système à ne détecter *que* les chutes). Or, la chute peut prendre des formes multiples (avant, arrière, latérale, droite ou avec rotation du corps, se terminant en position allongée ou assise, etc.).

Il est donc pratiquement impossible pour un capteur de chute de discriminer entre des vraies chutes et des fausses chutes de manière exhaustive. De plus, un même mouvement de chute, même le plus simple (par exemple la chute arrière après glissade) peut se dérouler de manière variable suivant le contexte et le sujet. Par conséquent il n'est pas possible d'atteindre une sensibilité totale et une spécificité totale avec des moyens de détection de chute raisonnables en termes d'encombrement, de complexité et de coût. De plus ce type d'équipement n'est pas apte à détecter une situation critique notamment due à un problème de santé lorsque la personne est allongée par exemple.

Le même type de problème peut se rencontrer avec un capteur de rythme cardiaque, où une situation peut être normale dans un certain contexte et alarmante dans un autre. Le document WO 2006/134041 divulgue, comme déclencheur d'alarme une situation où des valeurs de capteurs accélérométriques sont corrélées à l'observation d'une diminution lente du rythme cardiaque et de la température.

On sait par ailleurs améliorer encore la performance d'un capteur tel qu'un capteur de chute en combinant les informations qu'il délivre avec des informations de nature géographique ou temporelle (géolocalisation GPS, durée d'immobilisation, ...). On citera notamment le document EP 0 877 346. Toutefois, le système décrit dans ce document implique une certaine complexité technique et donc un coût conséquent.

La présente invention vise à pallier ces limitations de l'état de la technique et à proposer un procédé et un équipement qui, sans surcroît de complexité technique ou de coût, effectuent une discrimination améliorée, dictée par une stratégie décisionnelle basée sur la mesure de paramètres physiologiques afin de détecter une situation critique d'un sujet.

A cet effet, il est prévu selon l'invention un procédé pour détecter une situation critique d'un sujet par mesure d'un paramètre physiologique sur le sujet, caractérisé en ce qu'il comprend les étapes suivantes :

(a) mesurer le paramètre physiologique à l'aide d'un capteur embarqué sur le sujet,
(b) calculer une vitesse de variation du paramètre physiologique,
(c) déterminer une variable de synthèse pour le paramètre physiologique en prenant en compte à la fois la valeur du paramètre et sa vitesse de variation, et
(d) détecter une situation critique à partir de cette variable de synthèse.

Un tel procédé permet de détecter de manière fiable une situation critique sans pour autant accroître le coût et la complexité du dispositif, la partie décisionnelle étant réalisée par une unité de traitement de données déjà présente dans de tels dispositifs et ne nécessitant pas de matériel supplémentaire.

Avantageusement, mais facultativement, l'invention comporte au moins l'une des caractéristiques suivantes :

- le procédé comprend la mise en oeuvre des étapes (a) à (c) pour au moins deux paramètres physiologiques, et comprend en outre l'étape suivante :

(c') déterminer une variable décisionnelle globale à partir des différentes variables de synthèse, la situation critique étant détectée à l'étape (d) à partir de cette variable décisionnelle globale.

- les paramètres physiologiques sont choisis parmi les paramètres suivants : fréquence cardiaque, fréquence respiratoire, température du sujet, niveau d'activité du sujet, glycémie du sujet.
- la détermination de la variable décisionnelle globale est obtenue par une combinaison pondérée des variables de synthèse déterminées pour chacun des paramètres physiologiques.

- la détection d'une situation critique comprend la comparaison de la variable décisionnelle globale avec une ou plusieurs valeurs seuils.
- la détermination de la variable de synthèse pour un paramètre physiologique comprend une comparaison de la valeur du paramètre avec une ou plusieurs valeurs seuils.
- la détermination de la variable de synthèse pour un paramètre physiologique comprend la comparaison de la vitesse de variation dudit paramètre physiologique avec une ou plusieurs valeurs seuils.
- la valeur du paramètre est comparée avec deux valeurs seuils afin de déterminer si la valeur du paramètre est bas, normal ou haut, et la vitesse de variation du paramètre est comparée avec une valeur seuil afin de déterminer si la vitesse de variation du paramètre est faible ou forte.
- la valeur de la variable de synthèse est :

  o plus élevée si la valeur du paramètre est haute que si la valeur du paramètre est normale,
  o plus élevée si la valeur du paramètre est basse que si la valeur du paramètre est normale,
  o plus élevée si la vitesse de variation du paramètre est forte que si la vitesse de variation du paramètre est faible.

- le procédé comprend en outre l'étape suivante :

  (e) transmettre à distance par une liaison sans fil l'existence de ladite situation critique.

L'invention propose également un équipement de détection d'une situation critique d'un sujet, caractérisé en ce qu'il comprend :

- au moins un capteur embarqué apte à mesurer un paramètre physiologique, et
- une unité de traitement apte à recevoir les valeurs mesurées par le ou les capteurs et à calculer une vitesse de variation du ou de chacun des paramètres physiologiques, et à déterminer une variable de synthèse pour le ou chacun des paramètres physiologiques en prenant en compte, pour le paramètre ou pour au moins l'un des paramètres, à la fois sa valeur et sa vitesse de variation.

Des aspects préférés mais non limitatifs de cet équipement sont les suivants :

- l'unité de traitement est apte à exécuter le procédé tel que défini ci-dessus dans ses étapes (b), (c), (c') et (d).
- l'équipement est entièrement embarqué sur le sujet et comprend en outre un moyen de transmission à distance d'une information relative à l'existence d'une situation critique.
- l'équipement comprend un dispositif embarqué incluant les capteurs et une station externe abritant au moins une partie de l'unité de traitement et comprenant en outre un moyen de transmission à distance d'une information relative à l'existence d'une situation critique.

D'autres caractéristiques, buts et avantages de la présente invention apparaîtront à la lecture de la description détaillée qui va suivre, au regard des dessins annexés, donnés à titre d'exemples non limitatifs et sur lesquels :

- la figure 1 est une représentation schématique d'un équipement selon l'invention,
- la figure 2 est un diagramme schématique d'un procédé selon l'invention, et
- la figure 3 est un autre diagramme schématique d'un procédé selon l'invention.

Dans la description qui suit, on va décrire un système de surveillance à distance de personnes basé sur un ou plusieurs détecteurs embarqués sensibles aux mouvements du corps de la personne (notamment accéléromètres) et aux variables vitales de la personne (par exemple un capteur de rythme cardiaque, de rythme respiratoire, de pression artérielle, etc. et plus généralement de toute variable captée sur le corps humain et susceptible d'avoir une corrélation avec l'état de santé). Dans la suite du document, nous entendrons par « paramètres physiologiques » l'ensemble des variables mesurées par l'équipement de détection.

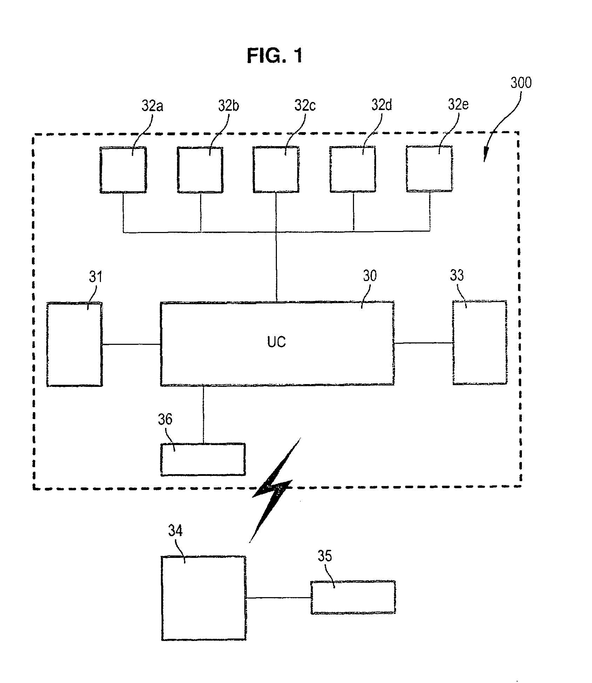

En référence à la figure 1, un équipement de détection de chute **300** selon l'invention comprend une unité centrale de traitement **30** associée à une mémoire **31,** l'ensemble étant par exemple réalisé sous forme d'un microcontrôleur et alimenté par une source d'alimentation embarquée telle qu'une batterie **36.** La mémoire **31** contient notamment le programme de détection tel qu'on va le décrire dans la suite, et les données associées. Un ensemble des capteurs embarqués **32a à 32e** sont connectés à des entrées de l'unité centrale **30,** chacun étant capable de mesurer un paramètre physiologique du sujet.

Par exemple, des capteurs d'inclinaison et des capteurs de mouvement peuvent être utilisés pour mesurer l'inclinaison et la composante de l'accélération de la pesanteur le long du tronc et d'une jambe de la personne. Ainsi par exemple, si la personne s'affaisse le long d'un mur et que son tronc reste vertical, le capteur placé sur les jambes pourra indiquer la chute de la personne.

Plus généralement, de tels capteurs **32a** à **32e** peuvent être prévus pour mesurer par exemple :

- la fréquence cardiaque,
- la fréquence respiratoire,
- la température du sujet, ou une relation entre la température du sujet et la température ambiante,
- le niveau d'activité du sujet (déterminé par exemple par les même capteurs que ceux utilisés pour détecter la chute),
- la glycémie, par exemple à l'aide d'un capteur de glucose disponible dans le commerce auprès de l'une des sociétés Medtronic, Abbott et Dexcom.

Un dispositif sonore **33** est connecté à l'unité centrale **30** pour que l'équipement puisse émettre un signal sonore d'alerte quand une chute est détectée. De la même manière, l'équipement de détection **300** est connecté via un dispositif émetteur non illustré, à une station externe **34,** qui est chargée de transmettre le signal d'alerte à un centre de télésurveillance **35** par voie hertzienne ou connexion téléphonique ou internet. Lorsqu'elle ne détecte pas de chute, l'unité centrale **30** peut fournir à intervalles de temps réguliers un signal de situation normale pour indiquer que l'équipement est en fonctionnement. L' unité centrale **30** peut aussi fournir à intervalles de temps réguliers ou non un signal indiquant que l'équipement est porté par la personne et que celle-ci est surveillée.

Dans une forme de réalisation, le traitement des mesures issues des capteurs est effectué au niveau de la station externe **34,** l'unité de contrôle **30** servant dans ce cas simplement à transmettre les mesures des capteurs **32a** à **32e** à la station externe **34.** Il peut être prévu dans ce cas que la station externe **34** possède un dispositif sonore destiné à émettre un signal sonore d'alerte plus puissant que celui de l'équipement de détection **300** en cas de chute détectée.

Ainsi, soit l'équipement est entièrement embarqué sur la personne et comprend un moyen de transmission à distance d'une information relative à l'existence d'une situation critique, soit l'équipement comprend un dispositif embarqué incluant les capteurs et une station externe abritant au moins une partie du calculateur et comprenant en outre un moyen de transmission à distance d'une information relative à l'existence d'une situation critique.

Selon l'invention, on détecte une situation critique d'un sujet portant l'équipement sur la base d'une combinaison des mesures du dispositif. A l'aide de cette combinaison, l'équipement peut détecter si une alerte doit être déclenchée ou non.

Plus particulièrement, on prévoit qu'une mesure d'un paramètre physiologique fournie par l'équipement de détection n'a pas nécessairement le même poids selon le paramètre physiologique, la valeur de ce paramètre et la vitesse de variation de ce paramètre.

Le procédé selon l'invention, mis en oeuvre par l'unité centrale **30** ou par la station externe **34** (ou encore de façon partagée entre elles) comprend ainsi les étapes suivantes :

- mesurer chacun des paramètres physiologiques,
- calculer une vitesse de variation de chacun des paramètres physiologiques,
- déterminer une variable de synthèse pour chacun des paramètres physiologiques en prenant en compte à la fois la valeur du paramètre et sa vitesse de variation,
- dans le cas où plusieurs paramètres physiologiques sont pris en compte, déterminer une variable décisionnelle globale pour l'ensemble de ceux-ci, et
- détecter une situation critique à partir de cette variable décisionnelle globale.

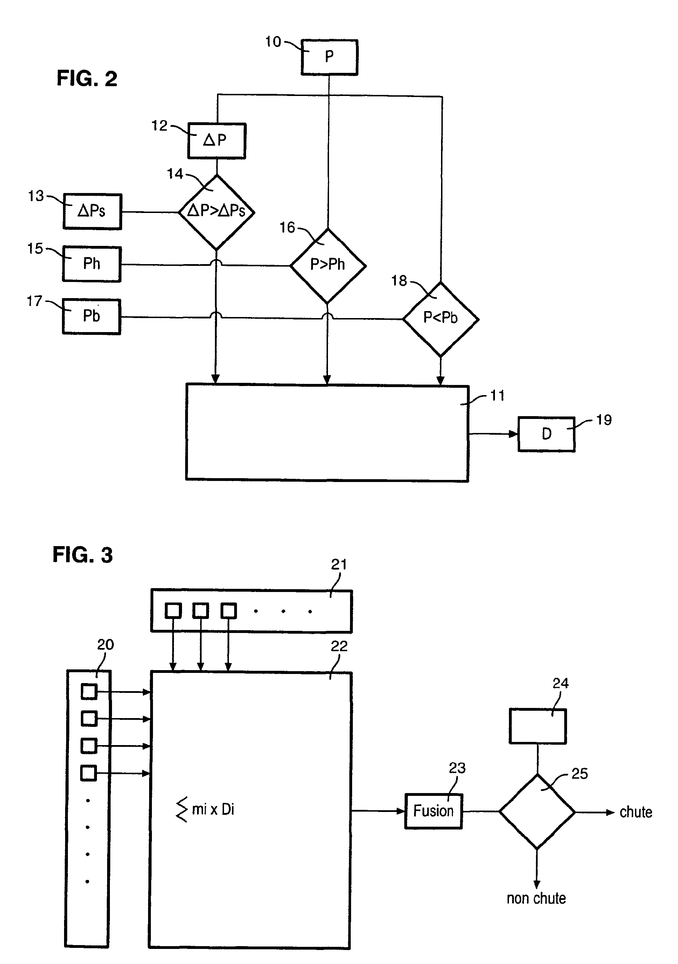

En référence à la figure 2, on va maintenant détailler un processus décisionnel selon l'invention. Chaque paramètre physiologique **p** est défini par sa valeur instantanée **p** et sa tendance **Δp** (valeur absolue de la variation de la valeur **p** entre deux temps de mesure séparés par un intervalle fixe). On prévoit avantageusement un processus de validation des mesures, par exemple en ne validant une mesure que si sa valeur est corrélée aux valeurs précédentes de cette mesure.

Deux valeurs seuils **ph** (haut) et **pb** (bas) sont définies pour chaque paramètre **p,** ainsi qu'une valeur seuil **Δps** pour la tendance **Δp.**

Ainsi, une comparaison est effectuée en **14** entre **Δp** et le seuil **Δps,** une comparaison est effectuée en **16** entre la valeur **p** et le seuil **ph,** et une comparaison est effectuée en **18** entre la valeur **p** et le seuil **pb.**

Par exemple, si le paramètre physiologique est la température corporelle (valeur normale 37°C), les trois valeurs seuils du paramètre peuvent être :

- **ph** =40°C

- **pb** =35°C
- Δ**ps** =1°C/heure

Ces seuils peuvent être déterminés, par l'expertise d'un ou plusieurs médecins qui s'appuieront sur leur connaissance de la physiologie humaine et de leur expérience praticienne. Ces seuils peuvent être également déterminés par une analyse statistique sur des données enregistrées sur un sujet (analyse intra-sujet) ou sur un groupe de sujets appartenant à une population cible (analyse inter-sujets). Cette analyse statistique peut être réalisée une seule fois ou bien périodiquement pour tenir compte de l'évolution du sujet ou de la population cible (variations saisonnières, tendances dues au vieillissement).

Avantageusement, certains de ces seuils, à la configuration du système, peuvent être ajustés en fonction de certains paramètres connus, physiologiques ou non, du sujet tels que son âge, son sexe, son indice massique, ses antécédents, son mode de vie, différents facteurs environnementaux, etc. Par exemple, pour une personne d'indice massique élevé, les valeurs de seuil de la glycémie seront plus élevées, les seuils de tendance de fréquence cardiaque et respiratoire seront plus élevés, etc.

Ces seuils variables peuvent être mis à jour périodiquement dans le cas où ces paramètres connus varient, de façon manuelle ou automatique dans le cas où le système est capable de connaître ces variations (âge par exemple).

A partir des résultats de ces comparaisons, le processus calcule une valeur de synthèse de la variable physiologique **D.** Cette valeur a pour but de fournir une information simplifiée de l'état du paramètre physiologique. Dans la suite du document, on considérera par convention que plus la valeur de synthèse **D** est haute, plus l'état de la variable physiologique parait inquiétant.

On peut comparer les valeurs d'un paramètre **p** par rapport à ses seuils **ph** et **pb** ainsi que sa tendance Δ**p** par-rapport au seuil Δ**ps** :

- Si **p** < pb, alors p est trop bas ;
- Si p > ph alors p est trop haut ;
- Si pb < p < ph alors p est normal ;
- Si Δp > Δ**ps** alors la tendance Δ**p** est forte, sinon elle est faible.

On élabore ensuite à l'étape **11** une matrice de synthèse pour le paramètre **p**, par exemple comme suit :

|  | Bas | Normal | Haut |
|---|---|---|---|
| Faible | 0.5 | 0 | 0.5 |
| Forte | 1 | 0.5 | 1 |

dans laquelle on affecte la valeur de synthèse la plus basse (D=0) si le niveau du paramètre et sa variation ne sont pas significatifs d'un trouble, et la valeur de synthèse la plus haute (D=1) si le paramètre s'écarte trop de la normale à la fois par sa valeur et sa tendance, et peut donc signifier un trouble.

Ainsi à partir d'une matrice de synthèse et des comparaisons effectuées aux étapes **14,16 et 18,** l'étape **11** fournit en sortie la valeur de synthèse **D.**

Par exemple si le paramètre physiologique est la fréquence cardiaque (valeur nominale 70 battements par minute ou BPM au repos pour une personne donnée) avec :

- ph =100 BPM
- pb =40 BPM
- Δps=15 BPM/minute

si à un instant donné, on enregistre p=60 BPM avec Δp=20 BPM/min, on affectera le paramètre p d'un niveau de synthèse D = 0,5.

A un instant donné on connaît les valeurs de n paramètres physiologiques :

$$\mathbf{<pi>} = [p1, p2,\ldots pi\ldots pn]$$

et leurs tendances :

$$\langle \mathbf{\Delta pi} \rangle = [\Delta p1, \Delta p2 ,\ldots \Delta pi\ldots\Delta pn]$$

Pour chacun de ces paramètres, des seuils phi et pbi sont fixés ainsi que les seuils de tendances $\Delta$**psi.**

On calcule donc les valeurs de synthèse $\langle \mathbf{Di} \rangle$ = [D1, D2,...Di...Dn]

- Comme on l'a indiqué, les paramètres pris en compte peuvent être notamment la fréquence cardiaque, la fréquence respiratoire, la température du sujet, ou une relation entre la température du sujet et la température ambiante, le niveau d'activité du sujet (déterminé par exemple par les accéléromètres) et la glycémie.

En référence à la figure 3, on affecte à chaque valeur de synthèse **Di** d'un paramètre **pi** un poids **mi**. Ainsi, dans le bloc **21**, un vecteur de poids **mi** $\langle \mathbf{mi} \rangle$ = [m1, m2 ,... mi...mn] est affecté, par exemple, tel que $\Sigma mi = 1$ (la somme de tous les poids est égale à 1) et dans le bloc **20**, un vecteur des valeur de synthèse di, $\langle \mathbf{Di} \rangle$ = [D1, D2 ,... Di...Dn], est affecté. Ces deux blocs sont liés au bloc **22**, dans lequel on calcule une valeur de fusion notée Fusion tel que par exemple :

$$\text{Fusion} = \Sigma mi^{*}Di$$

Cette valeur constitue une variable décisionnelle globale représentative de l'état des valeurs de synthèse des différents paramètres physiologiques, en tenant compte de leur importance grâce à la pondération ; cette information permet de décider si une situation critique est détectée ou non.

Ainsi, au niveau du bloc **25**, la valeur de fusion est comparée à une ou plusieurs valeurs seuil en vue de détecter une situation critique et d'enclencher ou non un signal d'alarme.

Par exemple, on peut établir par convention que si Fusion > 0,5 on est alors en présence d'une situation critique.

On peut envisager alternativement deux seuils sur la fusion, par exemple :

- Si 0,3 < Fusion < 0,7 on est en présence d'une situation préoccupante (alerte orange) ;
- et si 0,7 < Fusion on est en présence d'une situation critique (alerte rouge).

Voici un exemple concret avec pour paramètres la fréquence cardiaque (Fc en bat/mn), la fréquence respiratoire (Fr en nb/mn) et la température corporelle (T en °C) :

| paramètre | valeur mini | valeur maxi | seuil bas | seuil haut | seuil tendance | poids |
|---|---|---|---|---|---|---|
| Freq Cardiaque (Fc) | 50 | 180 | 60 | 90 | 13 | 0,5 |
| Respiration (Fr) | 1 | 10 | 4 | 7 | 0,9 | 0,3 |
| Température (T) | 35 | 42 | 36 | 39 | 0,7 | 0,2 |

| | | cas 1 | cas 2 | cas 3 |
|---|---|---|---|---|
| paramètre | Fc | 70 | 50 | 100 |
| seuil paramètre | | 0 | 1 | 1 |
| tendance | DFc | 5 | 5 | 15 |
| seuil tendance | | 0 | 0 | 1 |
| Décision Fc | D1 | 0 | 0,5 | 1 |
| | | | | |
| paramètre | Fr | 5 | 4 | 3 |
| seuil paramètre | | 0 | 0 | 1 |
| tendance | DFr | 0,5 | 1 | 1 |
| seuil tendance | | 0 | 1 | 1 |

(suite)

|  |  | cas 1 | cas 2 | cas 3 |
|---|---|---|---|---|
| Décisions Fr | D2 | 0 | 0,5 | 1 |
|  |  |  |  |  |
| paramètre | T | 36,4 | 35 | 41 |
| seuil paramètre |  | 0 | 1 | 1 |
| tendance | DT | 0,5 | 2 | 2 |
| seuil tendance |  | 0 | 1 | 1 |
| Décision T | D3 | 0 | 1 | 1 |
|  |  |  |  |  |
| **Fusion** |  | **0,00** | **0,60** | **1,00** |
| **alerte** |  | **non** | **orange** | **rouge** |

- dans le premier cas, il n'y a rien d'anormal.
- dans le cas 2, on relève un premier niveau d'alerte (orange) car la fréquence cardiaque est basse, la fréquence respiratoire est normale mais a tendance à s'abaisser, et la température corporelle est basse.
- dans le dernier cas, on va vers une tachycardie (fréquence cardiaque déjà haute et qui poursuit son accélération), au contraire la respiration basse ne suit pas ce rythme et on constate également un risque de foyer infectieux par la température élevée.

**Revendications**

1. Procédé pour détecter une situation critique d'un sujet par mesure d'un paramètre physiologique sur le sujet, comprenant les étapes suivantes :

   (a) mesurer le paramètre physiologique à l'aide d'un capteur embarqué sur le sujet (10),
   (b) calculer une vitesse de variation du paramètre physiologique (12),
   (c) déterminer une variable de synthèse pour le paramètre physiologique (19) en prenant en compte à la fois la valeur du paramètre et sa vitesse de variation, et
   (d) détecter une situation critique (25) à partir de cette variable de synthèse.

2. Procédé selon la revendication 1, comprenant la mise en oeuvre des étapes (a) à (c) pour au moins deux paramètres physiologiques, et comprend en outre l'étape suivante :

   (c') déterminer une variable décisionnelle globale (23) à partir des différentes variables de synthèse, la situation critique étant détectée à l'étape (d) à partir de cette variable décisionnelle globale.

3. Procédé selon la revendication 2, dans lequel les paramètres physiologiques sont choisis parmi les paramètres suivants : fréquence cardiaque, fréquence respiratoire, température du sujet, niveau d'activité du sujet, glycémie du sujet.

4. Procédé selon l'une des revendications 2 et 3, dans lequel la détermination de la variable décisionnelle globale est obtenue par une combinaison pondérée (21) des variables de synthèse déterminées pour chacun des paramètres physiologiques.

5. Procédé selon l'une des revendications 2 à 4, dans lequel la détection d'une situation critique comprend la comparaison de la variable décisionnelle globale (23) avec une ou plusieurs valeurs seuils (24).

6. Procédé selon l'une des revendications précédentes, dans lequel la détermination de la variable de synthèse pour un paramètre physiologique comprend une comparaison de la valeur du paramètre avec une ou plusieurs valeurs seuils (15,17).

**7.** Procédé selon l'une des revendications précédentes, dans lequel la détermination de la variable de synthèse pour un paramètre physiologique comprend la comparaison de la vitesse de variation dudit paramètre physiologique avec une ou plusieurs valeurs seuils (13).

**8.** Procédé selon les revendications 6 et 7 prises en combinaison, dans lequel la valeur du paramètre est comparée avec deux valeurs seuils afin de déterminer si la valeur du paramètre est bas, normal ou haut et en ce que la vitesse de variation du paramètre est comparée avec une valeur seuil afin de déterminer si la vitesse de variation du paramètre est faible ou forte.

**9.** Procédé selon la revendication 8, dans lequel la valeur de la variable de synthèse est :

- plus élevée si la valeur du paramètre est haute que si la valeur du paramètre est normale,
- plus élevée si la valeur du paramètre est basse que si la valeur du paramètre est normale,
- plus élevée si la vitesse de variation du paramètre est forte que si la vitesse de variation du paramètre est faible.

**10.** Procédé selon l'une des revendications 1 à 9, comprenant en outre l'étape suivante :

(e) transmettre à distance par une liaison sans fil l'existence de ladite situation critique.

**11.** Equipement de détection d'une situation critique d'un sujet, comprenant :

- au moins un capteur embarqué apte à mesurer un paramètre physiologique (10), et
- une unité de traitement apte à recevoir les valeurs mesurées par le ou les capteur et à calculer une vitesse de variation du ou de chacun des paramètres physiologiques (12), à déterminer une variable de synthèse pour le ou chacun des paramètres physiologiques (19) en prenant en compte, pour le paramètre ou pour au moins l'un des paramètres, à la fois sa valeur et sa vitesse de variation, et à détecter une situation critique à partir de cette variable de synthèse,

**12.** Equipement selon la revendication 11, comprenant aux moins deux capteurs embarqués aptes à mesurer au moins deux paramètres physiologiques différents, et dans lequel l'unité de traitement est apte à exécuter le procédé selon l'une des revendications 2 à 5.

**13.** Equipement selon la revendication 11 ou 12, lequel est entièrement embarqué sur le sujet et comprenant en outre un moyen de transmission à distance d'une information relative à l'existence d'une situation critique.

**14.** Equipement selon la revendication 11 ou 12, comprenant un dispositif embarqué incluant les capteurs et une station externe abritant au moins une partie de l'unité de traitement, et comprenant en outre un moyen de transmission à distance d'une information relative à l'existence d'une situation critique.

**Claims**

**1.** A process for detecting a critical situation of a subject by measuring a physiological parameter on the subject, comprising the following steps:

(a) measuring the physiological parameter by means of an embedded sensor on the subject (10),
(b) calculating a variation rate of the physiological parameter (12),
(c) determining a synthesis variable for the physiological parameter (19) by taking into account at the same time the value of the parameter and its variation rate, and
(d) detecting a critical situation (25) from this synthesis variable.

**2.** The process as claimed in Claim 1, comprising implementing steps (a) to (c) for at least two physiological parameters, and further comprising the following step:

(c') determining a global decision variable (23) from the different synthesis variables, the critical situation being detected in step (d) from this global decision variable.

**3.** The process as claimed in Claim 2, in which the physiological parameters are selected from the following parameters:

cardiac frequency, respiratory frequency, temperature of the subject, level of activity of the subject, glycaemia of the subject.

4. The process as claimed in any one of Claims 2 and 3, in which determination of the global decision variable is done by weighted combination (21) of the synthesis variables determined for each of the physiological parameters.

5. The process as claimed in any one of Claims 2 to 4, in which detection of a critical situation comprises comparison of the global decision variable (23) with one or more threshold values (24).

6. The process as claimed in any one of the preceding claims, in which determination of the synthesis variable for a physiological parameter comprises comparison of the value of the parameter with one or more threshold values (15, 17).

7. The process as claimed in any one of the preceding claims, in which determination of the synthesis variable for a physiological parameter comprises comparison of the variation rate of said physiological parameter with one or more threshold values (13).

8. The process as claimed in Claims 6 and 7 taken in combination, in which the value of the parameter is compared to two threshold values to determine if the value of the parameter is low, normal or high, and in that the variation rate of the parameter is compared to a threshold value to determine if the variation rate of the parameter is weak or strong.

9. The process as claimed in Claim 8, in which the value of the synthesis variable is:

- higher if the value of the parameter is high than if the value of the parameter is normal,
- higher if the value of the parameter is low than if the value of the parameter is normal,
- higher if the variation rate of the parameter is strong than if the variation rate of the parameter is weak.

10. The process as claimed in any one of Claims 1 to 9, further comprising the following step:

(e) remote transmitting via wireless link of the existence of said critical situation.

11. Equipment for detecting a critical situation of a subject, comprising:

- at least one embedded sensor capable of measuring a physiological parameter (10), and
- a processing unit capable of receiving the values measured by the sensor or the sensors and calculating a variation rate of the or of each of the physiological parameters (12), determining a synthesis variable for the or each of the physiological parameters (19) by taking into account its value and its variation rate at the same time, for the parameter or for at least one of the parameters, and detecting a critical situation from this synthesis variable.

12. Equipment as claimed in Claim 11, comprising at least two embedded sensors capable of measuring at least two different physiological parameters, and in which the processing unit is capable of executing the process as claimed in any one of Claims 2 to 5.

13. Equipment as claimed in Claim 11 or 12, which is fully embedded on the subject and further comprising remote transmission means of information relative to the existence of a critical situation.

14. Equipment as claimed in Claim 11 or 12, comprising an embedded device including the sensors and an external station housing at least part of the processing unit, and further comprising remote transmission means of information relative to the existence of a critical situation.

**Patentansprüche**

1. Verfahren zur Erkennung einer kritischen Situation einer Person durch Messung eines physiologischen Parameters an der Person, das die folgenden Schritte umfasst:

(a) Messung des physiologischen Parameters mit Hilfe eines an der Person (10) eingebetteten Sensors,
(b) Berechnung einer Änderungsgeschwindigkeit des physiologischen Parameters (12),
(c) Bestimmung einer Synthesevariablen für den physiologischen Parameter (19), indem gleichzeitig der Wert des Parameters und seine Änderungsgeschwindigkeit herangezogen werden, und
(d) Erkennung einer kritischen Situation (25) ausgehend von dieser Synthesevariablen.

2. Verfahren nach Anspruch 1, umfassend die Umsetzung der Schritte (a) bis (c) bei mindestens zwei physiologischen Parametern und umfassend ferner den folgenden Schritt:

(c') Bestimmung einer globalen Entscheidungsvariablen (23) ausgehend von verschiedenen Synthesevariablen, wobei die kritische Situation in Schritt (d) ausgehend von dieser globalen Entscheidungsvariablen erkannt wird.

3. Verfahren nach Anspruch 2, wobei die physiologischen Parameter unter folgenden Parametern gewählt sind: Herzfrequenz, Atemfrequenz, Temperatur der Person, Aktivitätsniveau der Person, Blutzucker der Person.

4. Verfahren nach einem der Ansprüche 2 und 3, wobei die Bestimmung der globalen Entscheidungsvariablen durch eine gewichtete Kombination (21) der für jeden der physiologischen Parameter bestimmten Synthesevariablen erhalten wird.

5. Verfahren nach einem der Ansprüche 2 bis 4, wobei die Erkennung einer kritischen Situation den Vergleich der globalen Entscheidungsvariablen (23) mit einem oder mehreren Schwellenwerten (24) umfasst.

6. Verfahren nach einem der vorhergehenden Ansprüche, wobei die Bestimmung der Synthesevariablen für einen physiologischen Parameter einen Vergleich des Wertes des Parameters mit einem oder mehreren Schwellenwerten (15, 17) umfasst.

7. Verfahren nach einem der vorhergehenden Ansprüche, wobei die Bestimmung der Synthesevariablen für einen physiologischen Parameter einen Vergleich der Veränderungsgeschwindigkeit des physiologischen Parameters mit einem oder mehreren Schwellenwerten (13) umfasst.

8. Verfahren nach den Ansprüchen 6 und 7 in Kombination, wobei der Wert des Parameters mit zwei Schwellenwerten verglichen wird, um zu bestimmen, ob der Wert des Parameters niedrig, normal oder hoch ist, und wobei die Veränderungsgeschwindigkeit des Parameters mit einem Schwellenwert verglichen wird, um zu bestimmen, ob die Veränderungsgeschwindigkeit des Parameters gering oder hoch ist.

9. Verfahren nach Anspruch 8, wobei der Wert der Synthesevariablen

- höher ist, wenn der Wert des Parameters hoch ist, als wenn der Wert des Parameters normal ist,
- höher ist, wenn der Wert des Parameters niedrig ist, als wenn der Wert des Parameters normal ist,
- höher ist, wenn die Veränderungsgeschwindigkeit des Parameters hoch ist, als wenn die Veränderungsgeschwindigkeit des Parameters gering ist.

10. Verfahren nach einem der Ansprüche 1 bis 9, das ferner den folgenden Schritt umfasst:

(e) Fernübertragung des Vorliegens der kritischen Situation durch eine drahtlose Verbindung.

11. Gerät zur Erkennung einer kritischen Situation einer Person, umfassend:

- mindestens einen eingebetteten Sensor, der zur Messung eines physiologischen Parameters (10) geeignet ist, und
- eine Auswerteeinheit, die dafür geeignet ist, die durch den oder die Sensor(en) gemessenen Werte zu empfangen und eine Veränderungsgeschwindigkeit des oder jedes der physiologischen Parameter (12) zu berechnen, eine Synthesevariable für den oder jeden der physiologischen Parameter (19) zu bestimmen, indem für den Parameter oder für mindestens einen der Parameter gleichzeitig sein Wert und seine Veränderungsgeschwindigkeit herangezogen werden, und ausgehend von dieser Synthesevariablen eine kritische Situation zu erkennen.

12. Gerät nach Anspruch 11, umfassend mindestens zwei eingebettete Sensoren, die dafür geeignet sind, mindestens zwei verschiedene physiologische Parameter zu messen, und wobei die Auswerteeinheit dafür geeignet ist, das Verfahren nach einem der Ansprüche 2 bis 5 durchzuführen.

13. Gerät nach Anspruch 11 oder 12, das vollständig an der Person eingebettet ist und ferner ein Mittel für die Fernübertragung einer Information über das Vorliegen einer kritischen Situation umfasst.

14. Gerät nach Anspruch 11 oder 12, umfassend eine eingebettete Vorrichtung, welche die Sensoren umfasst, und eine externe Station, die mindestens einen Teil der Auswerteeinheit aufnimmt, und umfassend ferner ein Mittel für die Fernübertragung einer Information über das Vorliegen einer kritischen Situation.

FIG. 1

**FIG. 2**

**FIG. 3**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2006134041 A
- EP 0877346 A